# EUROPÄISCHE PATENTSCHRIFT

(11) **EP 2 857 536 B1**
(45) Veröffentlichungstag und Bekanntmachung des Hinweises auf die Patenterteilung: **30.12.2015**
(21) Anmeldenummer: 13187287.1
(22) Anmeldetag: 03.10.2013
(51) Int. Cl.: C22C 23/00, A61F 2/28, C22C 23/04, C22F 1/06

(54) **Implantat für Patienten im Wachstum, Verfahren zu dessen Herstellung und Verwendung**
Implant for patients in growth, method for its preparation and use
Implant pour patients en cours de croissance, son procédé de fabrication et son utilisation

(43) Veröffentlichungstag der Anmeldung: 08.04.2015
(73) Patentinhaber: Weinberg, Annelie-Martina, 48465 Schüttorf (DE)
(72) Erfinder: Weinberg, Annelie-Martina, 48465 Schüttorf (DE)
(74) Vertreter: Jell, Friedrich

(56) Entgegenhaltungen:
- EP-A1- 1 840 235
- EP-A2- 2 511 390
- WO-A1-2009/148515
- WO-A2-2013/052791

## Beschreibung

Die Erfindung betrifft ein Implantat für Patienten im Wachstum, insbesondere für die Osteosynthese, mit einer Legierung auf Mg-Zn-Ca-Basis.

Aus dem Stand der Technik sind biodegradierbare Implantate bekannt (DE102009002709A1), deren Werkstoff aus einer Magnesiumlegierung besteht. Unter den Magnesiumlegierungen sind auch Legierungen auf Mg-Zn-Ca-Basis bekannt, deren Hauptlegierungselemente eine Biokompatibilität zum menschlichen Organismus aufweisen. Dadurch sind Reizerscheinungen an solch ein Implantat umgebenden Bereichen vermeidbar. Zudem eröffnet diese Biokompatibilität der Hauptlegierungselemente auch die Möglichkeit des Einsatzes von Implantaten, insbesondere Nägeln für die Osteosynthese, in der Kinder- und Jugendheilkunde, da die sich auflösenden Hauptlegierungselemente vom Organismus der Person im Wachstum aufgenommen und verwertet werden können. Des Weiteren weisen bekannte Nägel auf Mg-Zn-Ca-Basis aus Gründen der Festigkeit einen vergleichsweise hohen Zn-Gehalt auf, was allerdings zu einer verminderten Korrosionsfestigkeit des Nagels führt. Nebenlegierungselemente, insbesondere seltene Erden, können die Korrosionsfestigkeit des Nagels wiederum erhöhten, verringern jedoch dessen Biokompatibilität, womit die Verwendung derartig aufgebauter Nägel insbesondere in der Kinder- und Jugendheilkunde bedenklich ist.

Die Erfindung hat sich daher die Aufgabe gestellt, ein Implantat auf Mg-Zn-Ca-Basis der eingangs geschilderten Art derart zu verbessern, dass dieses hohe Festigkeit aufweist und dennoch höchsten Ansprüchen hinsichtlich seiner Biokompatibilität zum menschlichen Organismus, insbesondere eines Kindes, genügt.

Die Erfindung löst die gestellte Aufgabe hinsichtlich des Implantats dadurch, dass die Legierung 0,1 bis 0,6 Gew.-% Zink (Zn) und 0,2 bis 0,6 Gew.-% Calcium (Ca) und als Rest Magnesium (Mg) sowie herstellungsbedingt unvermeidbare Verunreinigungen mit jeweils maximal 0,01 Gew.-% und gesamt höchstens 0,1 Gew.-% aufweist, wobei der Quotient der Gewichtsprozente von Zn und Ca kleiner gleich 1 ist.

Weist die Legierung 0,1 bis 0,6 Gew.-% Zink (Zn) auf, kann zunächst durch diesen reduzierten Zn-Gehalt von einer erhöhten Korrosionsfestigkeit des Implantats ausgegangen werden. Andererseits ist durch den reduzierten Zn-Gehalt jedoch mit einer verminderten Mischkristallhärtung zu rechnen. Die Erfindung kann diesen Nachteil der dadurch reduzierten Fertigkeit verringern, indem die Legierung 0,2 bis 0,6 Gew.-% Calcium (Ca) aufweist, wobei der Quotient der Gewichtsprozente von Zn und Ca kleiner gleich 1 ist. Es hat sich nämlich herausgestellt, dass mit Hilfe dieser Grenzen in der Komposition der Legierung vornehmlich eine (Mg,Zn)2Ca-Phase (intermetallische Phase) ausgeschieden werden kann. Die Ausbildung anderer intermetallischer Phasen, wie MgZn2 und Mg6Zn3Ca2, können vorteilhaft zurückgedrängt werden, was sich einerseits als Vorteil in Bezug auf eine geringere Degradationsgeschwindigkeit des Implantats zeigen kann. Zusätzlich wirken in der erfindungsgemäßen Legierung die (Mg,Zn)2Ca-Teilchen als Hindernisse für Versetzungen, das heißt, es kommt auch zu Teilchenhärtung, was zu einer erheblichen Festigkeitssteigerung der Legierung beitragen kann. Die intermetallischen (Mg,Zn)2Ca-Ausscheidung hemmen aber auch in vorteilhafter Weise das Kornwachstum, was Festigkeit und Duktilität des Implantats erheblich verbessern kann. Da weiter diese (Mg,Zn)2Ca-Phase unedler als die ohnehin vergleichsweise unedle Mg-Matrix ist, wirken (Mg,Zn)2Ca-Phasen, welche die Korngrenze ,pinnen', nicht als kathodische Stellen und stören in weiterer Folge nicht einen gleichmäßigen Korrosionsangriff. Durch Letzteren wird auch eine besonders nachteilige Punktkorrosion vermieden, die zur Ausbildung von lokalen Stabilitätsproblemen am Implantat und damit für Bruchstellen mitverantwortlich ist. Das erfindungsgemäße medizinische Implantat kann damit auch eine über seine Biodegradation gleichmäßig abnehmende mechanische Festigkeit sorgen, um damit der angestrebten Therapie bzw. der Anwendung am Organismus zu entsprechen. Somit kann sich solch ein Implantat besonders für die Osteosynthese eignen. Aufgrund der vergleichsweise hohen Stabilität bzw. Festigkeit hinsichtlich Korrosion und mechanischer Belastungen kann es sich auch erübrigen, zusätzliche Legierungselemente wie beispielsweise seltene Erden zu verwenden. Es ist daher ausreichend, wenn das Implantat neben den Legierungselementen Zn, Ca als Rest Magnesium (Mg) sowie herstellungsbedingt unvermeidbare Verunreinigungen mit jeweils maximal 0,01 Gew.-% und gesamt höchstens 0,1 Gew.-% aufweist. Das erfindungsgemäße Implantat besteht somit praktisch ausschließlich aus biokompatiblen Elementen. Unter Anbetracht der genannten Vorteile kann sich dieses somit besonders für Patienten im Wachstum eignen.

Im Allgemeinen wird erwähnt, dass das Implantat für die Osteosynthese ein Krischner-Draht, eine Herbert-Schraube, ein Marknagel oder dergleichen sein kann. Vorzugweise ist das Implantat ein Nagel zur elastisch stabilen Markraumschienung (ESIN).

Besonders hohen Anforderungen an Stabilität bzw. Festigkeit hinsichtlich Korrosion und mechanischer Belastbarkeit kann die Legierung gerecht werden, indem diese in ihrer Mg-Matrix hinsichtlich der intermetallischen Phasen im Wesentlichen - also mehr als 50% - (Mg,Zn)2Ca-Ausscheidungsphasen enthält.

Vorgenanntes kann weiter verbessert werden, wenn die Legierung in ihrer Mg-Matrix ausschließlich (Mg,Zn)2Ca-Ausscheidungsphasen enthält.

Die Erfindung hat sich außerdem die Aufgabe gestellt, ein Verfahren zur Herstellung eines Implantats zu vereinfachen - und dennoch reproduzierbar für eine vergleichswese hohe chemische und mechanische Festigkeit zu sorgen. Zudem soll das Verfahren auch vergleichsweise kostengünstig durchführbar sein.

Die Erfindung löst die gestellte Aufgabe hinsichtlich des Verfahrens zur Herstellung eines Implantats für Patienten im Wachstum, insbesondere für die Osteosynthese, dadurch, dass dessen Legierung zur Ausbildung von (Mg,Zn)2Ca-Ausscheidungsphasen auf einer Temperatur im Bereich von 200 bis 400 Grad Celsius gehalten wird.

Wird dessen Legierung zur Ausbildung von (Mg,Zn)2Ca-Ausscheidungsphasen auf einer Temperatur im Bereich von 200 bis 400 Grad Celsius gehalten, kann das Wachstum der (Mg,Zn)2Ca-Ausscheidungsphasen gezielt beeinflusst werden, sodass besonders feinkörnige (Mg,Zn)2Ca-Ausscheidungsphasen gebildet werden. Damit kann auf verfahrenstechnisch einfach handhabbare und kostengünstige Weise ein medizinisches Implantat reproduzierbar mit hoher mechanischer Festigkeit hergestellt werden. Zudem kann das erfindungsgemäße Verfahren auch für in der Mg-Matrix gleichmäßig verteilte (Mg,Zn)2Ca-Ausscheidungsphasen sorgen, um am Implantat eine gleichmäßige Biodegradation zu gewährleisten. Je nach Dauer dieser Temperaturbehandlung kann so die mechanische und chemische Festigkeit der Legierung der angestrebten Therapie bzw. der Anwendung am Organismus entsprechend ausgelegt werden. Dies kann insbesondere bei einem medizinischen Implantat für die Osteosynthese, vor allem auch in der Kinder- und Jugendheilkunde, vorteilhaft sein, welches Implantat beispielsweise ein Krischner-Draht, eine Herbert-Schraube, ein Marknagel, ein Nagel zur elastisch stabilen Markraumschienung (ESIN) oder dergleichen sein ist. Im Allgemeinen wird festgehalten, dass diese Temperaturbehandlung in unterschiedlichsten Verfahrensschritten zur Herstellung des Implantats erfolgen kann. Beispielsweise vor einem Warmumformen des Rohteils, beim Warmumformen (z. B. durch Extrudieren, Schmieden, Walzen oder dergleichen) und/oder bei einer Wärmebehandlung (z. B. beim Glühen, Warmauslagern oder dergleichen). Die erfindungsgemäße Maßnahme ist daher besonders handhabungsfreundlich vorzusehen und vereinfacht das Verfahren erheblich.

Wird die Legierung auf einer Temperatur im Bereich von 200 bis 275 Grad Celsius gehalten, so kann sich in diesem Teilbereich die Ausbildung von (Mg,Zn)2Ca-Ausscheidungsphasen weiter erhöhen.

Wie bereits erwähnt, kann die Legierung zur bevorzugten Ausbildung von (Mg,Zn)2Ca-Ausscheidungsphasen angeregt werden, wenn vor der Warmformgebung die Legierung auf der Temperatur gehalten wird.

Vorzugsweise kann während oder vor und während der Warmformgebung die Legierung zur Ausbildung von (Mg,Zn)2Ca-Ausscheidungsphasen auf der Temperatur gehalten werden, um diese Wärmebehandlung mit der Formgebung zusammenzuführen und das Verfahren somit schneller und ökonomischer durchzuführen. Unter einem Warmumformen ist Extrudieren, Schmieden, Walzen oder dergleichen vorstellbar. Beispielsweise kann sich hierzu ein Schmieden des Implantat-Rohteils bei einer der Temperatur besonders auszeichnen, um mechanische und chemische Eigenschaften des Implantats zu optimieren. Nach seiner Warmformgebung kann das Implantat einer Endbearbeitung, beispielsweise einer spanenden Bearbeitung, unterworfen werden.

Zudem ist denkbar, die (Mg,Zn)2Ca-Ausscheidungsphasen dadurch vorzugsweise zu erhalten, indem während der Warmauslagerung die Legierung zur Ausbildung von (Mg,Zn)2Ca-Ausscheidungsphasen auf der Temperatur gehalten wird.

Besonders kann sich die Erfindung bei Verwendung als Werkstoff zur Herstellung eines Implantats für Patienten im Wachstum zur Anwendung bei der Osteosynthese auszeichnen, wenn eine Legierung auf Mg-Zn-Ca-Basis mit 0,1 bis 0,6 Gew.-% Zink (Zn), mit 0,2 bis 0,6 Gew.-% Calcium (Ca) und als Rest Magnesium (Mg) sowie herstellungsbedingt unvermeidbaren Verunreinigungen mit jeweils maximal 0,01 Gew.-% und gesamt höchstens 0,1 Gew.-%, wobei der Quotient der Gewichtsprozente von Zn und Ca kleiner gleich 1 ist.

Insbesondere kann sich die Verwendung des Werkstoff für einen Krischner-Draht, eine Herbert-Schraube, einen Marknagel und/oder einen Nagel zur elastisch stabilen Markraumschienung eignen.

Zur Dokumentation der erzielten Effekte wurden als medizinisches Implantat Nägel mit unterschiedlichen Legierungen auf Mg-Zn-Ca-Basis hergestellt. Die Zusammensetzungen der untersuchten Legierungen sind in der Tabelle 1 angeführt.

**Tabelle 1: Übersicht zu den Nägeln**

| Nagel-Nr. | Zusammensetzung |
|---|---|
| 1 | MgZn1,Ca0,25Mn0,15Y2 |
| 2 | MgZn5,Ca0,25Mn0,15 |
| 3 | MgZn0,4Ca0,4 |

Beim Nagel Nr. 1 der Tabelle 1 handelt es sich um eine bekannte Magnesiumlegierung unter Verwendung seltener Erden Y als Legierungselement. Der ebenso aus dem Stand der Technik bekannte Nagel Nr. 2 nach Tabelle 1 verzichtet auf seltene Erden als Legierungselemente, benötigt zum Erreichen der gewünschten Festigkeit allerdings einen erhöhten Anteil an Zn, was die chemische Festigkeit (Korrosionsfestigkeit) des Nagels vermindert. Der in Tabelle 1 in seiner Zusammensetzung beispielsweise angegebene Nagel Nr. 3 weist die erfindungsgemäße Magnesiumlegierung auf. Dieser Nagel Nr. 3 wurde aus einem abgekühlten, extrudierten Rohteil mit nachfolgender spanenden Bearbeitung hergestellt. Die Extrusion erfolgte innerhalb der Temperaturgrenzen von 200 bis 400 °C.

Die genannten Nägel wurden auf ihre chemischen und mechanischen Festigkeiten hin untersucht. Hierzu wurden die Zugfestigkeit Rm, die Streckgrenze Rp0,2 und die Bruchdehnung A5 im Zugversuch bestimmt. Zudem wurde die Degradation in SBF (simulierte Körperflüssigkeit) gemessen. Die erhaltenen Messwerte sind in der Tabelle 2 zusammengefasst.

**Tabelle 2: Messergebnisse der untersuchten Nägel**

| Nagel-Nr. | R_{p0,2} [MPa] | Rₘ[MPa] | A₅ | SBF [mm/Jahr] |
|---|---|---|---|---|
| 1 | 150 | 250 | 20 | 0,5 |
| 2 | 210 | 295 | 18 | 4 |
| 3 | 200 | 250 | 22 | 0,25 |

Der gegenüber Nagel Nr. 2 vergleichsweise niedrige Zinkgehalt des Nagels Nr. 3 führt, wie der Tabelle 2 zu entnehmen, zu keinen Nachteilen in der mechanischen Festigkeit, profitiert dadurch jedoch ganz besonders in einer erhöhten chemischen Festigkeit von höchstens 0,25 mm/Jahr Degradation. Dies ist selbst niedriger als die gemessene Degradation am Nagel Nr. 1, der nachteilig seltene Erden in seiner Legierung aufweist.

Ein standfestes biodegradierbares Implantat ist somit geschaffen, das sich besonders für die Kinder- und Jugendheilkunde bzw. allgemein für Patienten im Wachstum eignet. Dies wird gewährleistet, da keine seltenen Erden Verwendung finden, die Legierungsbestandteile also biokompatibel und so vom wachsenden Organismus verwertbar sind - zusätzlich aber trotzdem hohe mechanische und chemische Festigkeit zur Verfügung gestellt werden.

## Patentansprüche

1. Implantat für Patienten im Wachstum, insbesondere für die Osteosynthese, mit einer Legierung auf Mg-Zn-Ca-Basis, **dadurch gekennzeichnet, dass** die Legierung
0,1 bis 0,6 Gew.-% Zink (Zn) und
0,2 bis 0,6 Gew.-% Calcium (Ca)
und als Rest Magnesium (Mg) sowie herstellungsbedingt unvermeidbare Verunreinigungen mit jeweils maximal 0,01 Gew.-% und gesamt höchstens 0,1 Gew.-% aufweist, wobei der Quotient der Gewichtsprozente von Zn und Ca kleiner gleich 1 ist.

2. Implantat nach Anspruch 1, **dadurch gekennzeichnet, dass** die Legierung in ihrer Mg-Matrix hinsichtlich der intermetallischen Phasen im Wesentlichen (Mg,Zn)2Ca-Ausscheidungsphasen enthält.

3. Implantat nach Anspruch 2, **dadurch gekennzeichnet, dass** die Legierung in ihrer Mg-Matrix ausschließlich (Mg,Zn)2Ca-Ausscheidungsphasen enthält.

4. Verfahren zur Herstellung eines Implantats für Patienten im Wachstum, insbesondere für die Osteosynthese, nach Anspruch 1, 2 oder 3, bei dem dessen Legierung zur Ausbildung von (Mg,Zn)2Ca-Ausscheidungsphasen auf einer Temperatur im Bereich von 200 bis 400 Grad Celsius gehalten wird.

5. Verfahren nach Anspruch 4, **dadurch gekennzeichnet, dass** die Legierung zur Ausbildung von (Mg,Zn)2Ca-Ausscheidungsphasen auf einer Temperatur im Bereich von 200 bis 275 Grad Celsius gehalten wird.

6. Verfahren nach Anspruch 4 oder 5, **dadurch gekennzeichnet, dass** vor der Warmformgebung die Legierung zur Ausbildung von (Mg,Zn)2Ca-Ausscheidungsphasen auf der Temperatur gehalten wird.

7. Verfahren nach Anspruch 4, 5 oder 6, **dadurch gekennzeichnet, dass** während der Warmformgebung die Legierung zur Ausbildung von (Mg,Zn)2Ca-Ausscheidungsphasen auf der Temperatur gehalten wird.

8. Verfahren nach einem der Ansprüche 4 bis 7, **dadurch gekennzeichnet, dass** während der Warmauslagerung die Legierung zur Ausbildung von (Mg,Zn)2Ca-Ausscheidungsphasen auf der Temperatur gehalten wird.

9. Verwendung einer Legierung auf Mg-Zn-Ca-Basis mit 0,1 bis 0,6 Gew.-% Zink (Zn), mit 0,2 bis 0,6 Gew.-% Calcium (Ca) und als Rest Magnesium (Mg) sowie herstellungsbedingt unvermeidbaren Verunreinigungen mit jeweils maximal 0,01 Gew.-% und gesamt höchstens 0,1 Gew.-%, wobei der Quotient der Gewichtsprozente von Zn und Ca kleiner gleich 1 ist, als Werkstoff zur Herstellung eines Implantats für Patienten im Wachstum zur Anwendung bei der Osteosynthese.

10. Verwendung nach Anspruch 9, **dadurch gekennzeichnet, dass** das Implantat als Krischner-Draht, Herbert-Schraube, Marknagel und/oder Nagel zur elastisch stabilen Markraumschienung verwendet wird.

## Claims

1. Implant for growing patients, in particular for osteosynthesis, comprising an alloy based on Mg-Zn-Ca, **characterized in that** the alloy comprises
0.1 to 0.6 % by weight zinc (Zn) and
0.2 to 0.6 % by weight calcium (Ca)
and magnesium (Mg) as the remainder, as well as unavoidable impurities caused by production in a maximum proportion of 0.01% by weight each and at most 0.1% by weight in total, wherein the quotient of the percentages by weight of Zn and Ca being less than or equal to 1.

2. Implant according to claim 1, **characterized in that** the alloy contains, in terms of intermetallic phases, substantially (Mg,Zn)2Ca precipitation phases in its Mg matrix.

3. Implant according to claim 2, **characterized in that** the alloy contains exclusively (Mg,Zn)2Ca precipitation phases in its Mg matrix.

4. Method for producing an implant for growing patients, in particular for osteosynthesis, according to claim 1, 2 or 3, in which the alloy thereof is kept at a temperature in the range from 200 to 400 degrees Celsius in order to form (Mg,Zn)2Ca precipitation phases.

5. Method according to claim 4, **characterized in that** the alloy is kept at a temperature in the range from 200 to 275 degrees Celsius in order to form (Mg,Zn)2Ca precipitation phases.

6. Method according to claim 4 or 5, **characterized in that**, prior to the hot forming, the alloy is kept at the temperature in order to form (Mg,Zn)2Ca precipitation phases.

7. Method according to claim 4, 5 or 6, **characterized in that**, during the hot forming, the alloy is kept at the temperature in order to form (Mg,Zn)2Ca precipitation phases.

8. Method according to any of claims 4 to 7, **characterized in that**, during the artificial ageing, the alloy is kept at the temperature in order to form (Mg,Zn)2Ca precipitation phases.

9. Use of an alloy based on Mg-Zn-Ca comprising 0.1 to 0.6% by weight zinc (Zn), 0.2 to 0.6% by weight calcium (Ca) and magnesium (Mg) as the remainder, as well as unavoidable impurities caused by production in a maximum proportion of 0.01% by weight each and at most 0.1% by weight in total, wherein the quotient of the percentages by weight of Zn and Ca being less than or equal to 1, as a material for producing an implant for growing patients for application in osteosynthesis.

10. Use according to claim 9, **characterized in that** the implant is used as a Kirschner wire, Herbert screw, intramedullary nail and/or nail for elastic stable intramedullary nailing.

## Revendications

1. Implant pour des patients en période de croissance, en particulier pour une ostéosynthèse, avec un alliage à base de Mg-Zn-Ca, **caractérisé en ce que** l'alliage contient
0,1 à 0,6 % en poids de zinc (Zn) et
0,2 à 0,6 % en poids de calcium (Ca)
et du magnésium (Mg) pour le reste ainsi que des impuretés inévitables liées à la fabrication représentant chacune 0,01 % en poids et au maximum 0,1 % en poids au total, le quotient des pourcentages en poids de Zn et Ca étant inférieur ou égal à 1.

2. Implant selon la revendication 1, **caractérisé en ce que** l'alliage contient pour l'essentiel dans sa matrice de magnésium, en ce qui concerne les phases intermétalliques, des phases de précipitation de (Mg,Zn)2Ca.

3. Implant selon la revendication 2, **caractérisé en ce que** l'alliage contient exclusivement des phases de précipitation de (Mg,Zn)2Ca dans sa matrice de magnésium.

4. Procédé pour la fabrication d'un implant pour des patients en période de croissance, en particulier pour une ostéosynthèse, selon la revendication 1, 2 ou 3, dans lequel l'alliage de celui-ci est maintenu à une température comprise entre 200 et 400 degrés Celsius afin de former des phases de précipitation de (Mg,Zn)2Ca.

5. Procédé selon la revendication 4, **caractérisé en ce que** l'alliage est maintenu à une température comprise entre 200 et 275 degrés Celsius afin de former des phases de précipitation de (Mg,Zn)2Ca.

6. Procédé selon la revendication 4 ou 5, **caractérisé en ce que** l'alliage est maintenu à température afin de former des phases de précipitation de (Mg,Zn)2Ca avant le formage à chaud.

7. Procédé selon la revendication 4, 5 ou 6, **caractérisé en ce que** l'alliage est maintenu à température afin de former des phases de précipitation de (Mg,Zn)2Ca pendant le formage à chaud.

8. Procédé selon l'une des revendications 4 à 7, **caractérisé en ce que** l'alliage est maintenu à température afin de former des phases de précipitation de (Mg,Zn)2Ca pendant la précipitation à chaud.

9. Utilisation d'un alliage à base de Mg-Zn-Ca avec 0,1 à 0,6 % en poids de zinc (Zn), avec 0,2 à 0,6 % en poids de calcium (Ca) et du magnésium (Mg) pour le reste ainsi que des impuretés inévitables liées à la fabrication représentant chacune 0,01 % en poids et au maximum 0,1 % en poids au total, dans lequel le quotient des pourcentages en poids de Zn et Ca est inférieur ou égal à 1, comme matériau pour la fabrication d'un implant destiné à être utilisé pour des patients en phase de croissance lors d'une ostéosynthèse.

10. Utilisation selon la revendication 9, **caractérisée en ce que** l'implant est utilisé sous la forme d'une broche de Kirschner, d'une vis de Herbert, d'un clou centromédullaire ou d'un clou de Prévot.
